Europäisches Patentamt

(19) European Patent Office

Office européen des brevets

(11) Numéro de publication : **0 071 515**
**B1**

(12) **FASCICULE DE BREVET EUROPÉEN**

(45) Date de publication du fascicule du brevet :
30.10.85

(51) Int. Cl.⁴ : **A 61 K 39/02**, A 61 K 39/09,
A 61 K 39/102,
A 61 K 39/108, C 12 P 19/04

(21) Numéro de dépôt : 82401343.7

(22) Date de dépôt : 19.07.82

(54) Procédé d'obtention de polyosides capsulaires, polyosides capsulaires ainsi obtenus et leur application à la préparation de vaccins.

(30) Priorité : 30.07.81 FR 8114842

(43) Date de publication de la demande :
09.02.83 Bulletin 83/06

(45) Mention de la délivrance du brevet :
30.10.85 Bulletin 85/44

(84) Etats contractants désignés :
AT BE CH DE FR GB IT LI LU NL SE

(56) Documents cités :
EP-A- 0 001 945
EP-A- 0 002 404
EP-A- 0 017 322
EP-A- 0 030 166
US-A- 2 166 963
US-A- 2 528 972
US-A- 4 264 764
US-A- 4 307 080
THE JOURNAL OF IMMUNOLOGY, vol. 110, no. 1, janvier 1973, pages 262-268, New York (USA); D.L. KASPER et al.: "Immunochemical similarity between polysaccharide antigens of Escherichia coli 07:K1(L): NM and group B Neisseria meningitidis"
UNLISTED DRUGS, vol. 28, no. 12, décembre 1976, page 205, New York (USA);
UNLISTED DRUGS, vol. 15, no. 11, novembre 1963, page 84, New York (USA);
MICROBIOLOGY ABSTRACTS, vol. 7, no. 6, juin 1972, section B, no. 5070, page 165, Boston, Mass. (USA); P. ANDERSON ET AL: "Immunization of humans with polyribophosphate, the capsular antigen of Hemophilus influenzae, type b »

(73) Titulaire : BERRI-BALZAC Société dite:
42, Rue Rouget-de-Lisle
F-92151 Suresnes Cedex (FR)

(72) Inventeur : Yavordios, Demètre INSTITUT DE RECHERCHE
SCIENTIFIQUE "I.R.S." Avenue Foch
F-01400 Chatillon sous Chalaronne (FR)
Inventeur : Cousin, Mireille INSTITUT DE RECHERCHE
SCIENTIFIQUE "I.R.S." Avenue Foch
F-01400 Chatillon sous Chalaronne (FR)

(74) Mandataire : Polus, Camille et al
c/o Cabinet Lavoix 2, Place d'Estienne d'Orves
F-75441 Paris Cedex 09 (FR)

Jouve, 18, rue St-Denis, 75001 Paris, France

février 1974, pages 649-55, (USA) ; M. ARGAMAN et al. : " Polyribitol-phosphate : an antigen of four gram-positive bacteria cross-reactive with the capsular polysaccharide of Haemophilus influenzae type b " BIOCHEMISTRY, vol. 3, no. 10, 25 octobre 1964, pages 1548-1550, New York (USA) ; M. HEIDELBERGER et al. : " The specific polysaccharide of type XVIIIA Pneumococcus "
Marchal et al. " Milieux de culture et identification biochimique des bactéries ", Ed. DOIN, Paris 1973, p. 8
Précis de microbiologie, Tome I, Ed. Masson, pp. 62-65

## Description

La présente invention concerne un procédé d'obtention de polyosides capsulaires, les polyosides obtenus par ce procédé et l'application de ces polyosides à la préparation de vaccins.

On sait que la lutte contre les maladies infectieuses peut être exercée à deux niveaux : soit par une action directe à l'égard du germe pathogène (antibiotiques, antiseptiques), soit par une action indirecte par le renforcement de défenses de l'organisme (vaccination, sérothérapie, immunomodulation).

L'utilisation des agents susceptibles d'agir directement se heurte souvent à certaines difficultés pouvant limiter son action (notamment toxicité de la molécule et résistance bactérienne).

Par contre, le renforcement des défenses de l'organisme par l'acquisition des anticorps spécifiques présente des avantages certains, notamment du point de vue de la durée de la protection et de la spécificité. Mais, la vaccination à l'aide de corps microbiens entiers n'est pas toujours sans inconvénients : phénomènes d'hypersensibilisation, mauvaise tolérance ou pyrogénicité sont parmi les plus fréquents. Ils sont liés à la présence de multiples antigènes de la bactérie.

C'est donc tout naturellement vers l'isolement de l'antigène responsable des anticorps protecteurs que les efforts des chercheurs se sont cristallisés.

Dans le cas des bactéries capsulées, les polyosides capsulaires se révèlent immunogènes chez l'homme, avec peu de réactions secondaires.

Une première application chez l'homme de ces polyosides capsulaires est l'apparition du vaccin antiméningococcique A et C et, tout récemment, du vaccin polyvalent contenant les polyosides purifiés de quatorze types sérologiques de pneumocoque.

Plusieurs autres bactéries capsulées sont impliquées dans divers processus pathologiques, par exemple :
— Hemophilus influenzae, type b,
— Klebsiella pneumoniae,
— Escherichia coli, ou
— Streptocoques, groupe B,
et des vaccins correspondants peuvent être préparés à partir de leurs polyosides capsulaires.

Dans la demande de brevet européen 0 002 404, on a décrit un procédé d'obtention de polyosides capsulaires à partir de Streptococcus pneumoniae.

Selon ce procédé, on cultive le microorganisme à l'aide de milieux de culture complexes. Puis, après inactivation au phénol, sans séparer les microorganismes du milieu de culture, on effectue une première addition d'alcool tel que l'éthanol, on sépare les impuretés précipitées, on effectue une deuxième addition d'alcool afin de précipiter alors le polyoside. Le polyoside remis en suspension est ensuite débarrassé des substances contaminantes (protéines, acides nucléiques) par un traitement enzymatique ou par un traitement par un surfactif cationique (bromure de citrimonium), qui peuvent être complétés par une diafiltration.

Ce procédé n'a pas, en fait, de caractère général, car les conditions spécifiques de chaque stade varient avec le polyoside à séparer. Ainsi, il est nécessaire de faire des pré-tests afin de déterminer les conditions des diverses précipitations. De plus, en raison de l'utilisation de certains milieux, ce procédé est susceptible d'entraîner une dégradation des chaînes polyosidiques.

La présente invention vise à fournir un procédé plus général, plus simple et moins dégradant permettant d'obtenir des polyosides de masse moléculaire très élevée.

La présente invention a pour objet un procédé d'obtention de polyosides capsulaires immunogènes à partir de bactéries capsulaires les contenant, caractérisé en ce que l'on effectue une culture des bactéries en milieu synthétique pouvant contenir jusqu'à environ 0,5 % en poids de produits d'origine naturelle, à la fin de la phase de croissance de cette culture on sépare les corps microbiens, on soumet la phase liquide débarrassée des corps microbiens à une séparation par filtration sur une membrane retenant les molécules d'une masse moléculaire égale ou supérieure à 100 000 daltons, obtenant ainsi un rétentat riche en polyosides capsulaires, et l'on élimine les protéines, les acides nucléiques et les lipides de ce rétentat obtenant ainsi un polyoside capsulaire purifié.

Ainsi, selon une caractéristique essentielle de la présente invention, on effectue des bactéries en milieu synthétique. Par milieu synthétique, on désigne des solutions aqueuses de composés chimiques connus, de faible masse moléculaire qui sont présents en proportions déterminées et pouvant contenir jusqu'à environ 0,5 % en poids de produits d'origine naturelle, tels que des hydrolysats de protéine.

En outre, selon la présente invention, l'extraction des polyosides est effectuée sur le surnageant de la culture débarrassée des corps microbiens et non pas sur l'ensemble du milieu de culture.

La filtration sur une membrane dont la porosité est capable de retenir les molécules d'une masse moléculaire égale ou supérieure à 100 000 daltons permet d'obtenir rapidement un rétentat renfermant principalement les polyosides, ainsi que, en faibles proportions, des protéines, des acides nucléiques et des lipides.

L'élimination des contaminants est avantageusement effectuée de la façon suivante : on soumet le rétentat à une hydrolyse enzymatique, notamment à l'aide d'un mélange de Pronase, RNase et DNase, on ajoute un mélange butanol-chloroforme (de préférence un mélange 1 : 1 en volume), on sépare la phase aqueuse, on soumet la phase aqueuse à une dialyse et on effectue une filtration sur une membrane retenant les molécules ayant une masse moléculaire égale ou spérieure à 100 000 daltons.

Le polyoside purifié peut être séparé, de manière connue, du rétentat par précipitation à l'aide d'un alcool tel que l'éthanol.

Les exemples suivants illustrent la présente demande :

## Exemple 1

Polyoside pneumococcique du type 3.

On part d'une souche lyophilisée de Streptococcus pneumoniae de type 3.

a) Préparation de lots d'ensemencement.
On introduit la souche lyophilisée dans du bouillon T.
On effectue la culture pendant 16 heures à 37 °C.
On mélange 5 ml de la culture obtenue avec 15 ml de lait écrémé stérilisé. On distribue dans des ampoules à raison de 0,5 ml/ampoule et on lyophilise. Les lyophilisats sont conservés à + 4 °C.

b) Préculture.
On introduit un lot d'ensemencement (lyophilisat en ampoule) dans un ballon contenant 200 ml du milieu semi-synthétique indiqué ci-après et on effectue la culture à 37 °C pendant 6 heures.

| Milieu de culture : | | |
|---|---:|---|
| Peptone pancréatique de caséine (IBF) | 1 500 | mg |
| l-cystine | 150 | mg |
| dl-tryptophane | 20 | mg |
| l-tyrosine | 200 | mg |
| Phosphate bipotassique | 4 960 | mg |
| d-glucose anhydre | 12 500 | mg |
| Sulfate de magnésium, 7H$_2$O | 500 | mg |
| Sulfate ferreux, 7H$_2$O | 5 | mg |
| Sulfate de zinc, 7H$_2$O | 0,8 | mg |
| Sulfate de manganèse, 7H$_2$O | 0,3 | mg |
| Acide chlorhydrique RP fumant | 17,8 | mg |
| d-biotine | 15 | mg |
| Acide nicotinique | 1 | mg |
| Pyridoxine HCl | 1 | mg |
| Riboflavine | 1 | mg |
| Thiamine | 1 | mg |
| Pentothénate de calcium | 5 | mg |
| Adénine HCl | 10 | mg |
| Uracile | 10 | mg |
| Chlorure de choline | 10 | mg |
| Asparagine | 100 | mg |
| Eau distillée q.s.p. | 1 000 | ml |

c) Culture.
La culture proprement dite est effectuée dans un fermenteur contenant 18 l du milieu de culture défini sous b. On ensemence à raison de 1 % avec la préculture.
La culture est effectuée à 37 °C, à pH 7,4, sous agitation pendant généralement environ 16 h, jusqu'à la fin de la phase exponentielle de croissance.

d) Séparation du surnageant.
A la fin de la phase exponentielle, on sépare immédiatement le surnageant par centrifugation en continu à 5 000 t/min (3 000 g). Le surnageant est soumis à une filtration stérilisante sur membrane Millipore® 0,22 µm et utilisé immédiatement ou bien il est conservé à − 25 °C.

e) Extraction.
Un volume de surnageant égal à environ 20 litres est concentré à un volume de 2 litres par filtration sur membrane Millipore® 10$^5$, permettant de retenir les molécules d'une masse moléculaire égale ou supérieure à 100 000 daltons.
Le rétentat ainsi obtenu est diafiltré sur membrane Diaflo® 10$^5$ après addition de 5 litres d'eau distillée stérile et apyrogène pour compléter la séparation selon la masse moléculaire.
Plusieurs diafiltrations successives permettent de concentrer à environ 350 ml qui sont lyophilisés dans des ballons de 1 litre.
Toutes ces opérations sont effectuées à une température au plus égale à 8 °C.

4

f) Purification.

Le produit intermédiaire lyophilisé est dissous dans 900 ml de tampon phosphate 0,01 M-pH 7.

On ajoute ensuite 20 mg de RNase type 1, 20 mg de DNase pancréatique et 180 µl (20 unités/ml) de RNase T1. On effectue une incubation de 4 h à 37 °C. On ajoute alors 170 mg de Pronase type 8, 900 µl de phénol pur et 2 gouttes de toluène.

On incube le mélange pendant une nuit à 37 °C. On arrête la réaction et on ajoute un volume égal (900 ml) d'un mélange butanol-chloroforme 1 : 1. On agite pendant 1 h. On décante les deux phases par centrifugation à 10 000 t/min (12 000 g) pendant 30 min.

La phase aqueuse qui contient le polyoside est dialysée contre 20 litres d'eau distillée stérile et apyrogène pendant environ 60 h.

On diafiltre le dialysat sur membrane Amicon® $10^5$. Le volume du rétentat est de 1 140 ml. On ajoute 160 ml d'acétate de sodium 40 % (concentration finale 5 %) et 3,9 litres d'éthanol absolu (3 volumes).

On recueille le précipité par centrifugation à 12 000 t/min. (17 000 g) pendant 30 min. On dissout ce précipité dans de l'eau distillée apyrogène. On répartit la solution en flacons sous un volume de 4 ml. On lyophilise sous vide. On obtient 9 g de produit. C'est le polysaccharide purifié qui contient :

| | |
|---|---|
| — sucres | 64 % |
| — protéines | 0,8 % |
| — acide nucléique | < 0,8 % |

Toutes les opérations (en dehors des incubations) sont effectuées entre 0 et + 4 °C.

## Exemple 2

Polyoside pneumococcique du type 23.

On part d'une souche lyophilisée de Streptococcus pneumoniae du type 23.

On effectue la culture et la séparation du surnageant dans les conditions définies à l'exemple 1 (stades a à d).

L'extraction et la purification du polyoside sont effectuées de la façon suivante :

e) Extraction

Un volume de surnageant égal à 15 litres est concentré à un volume de 1,5 litre dans les conditions définies à l'exemple 1-e.

Trois diafiltrations successives permettent de concentrer, puis de lyophiliser. On obtient 1,8 g de produit ayant la composition suivante :

| | |
|---|---|
| — sucres | 60 % |
| — protéines | 19 % |
| — acide nucléique | 3,6 % |

f) Purification

On reprend le produit intermédiaire lyophilisé avec 200 ml de tampon phosphate 0,01 M — pH 7. On ajoute 1 mg de RNase, 1 mg de DNase et 20 unités de RNase Tl/ml. On incube pendant 3 h à 37 °C. On ajoute de la Pronase type 8 (180 µg/ml), 1 µl/ml de phénol et une goutte de toluène. On incube pendant une nuit à 37 °C.

On effectue trois extractions successives avec un mélange à volume égal (200 ml) de butanol-chloroforme 1 : 1. Le mélange est agité mécaniquement.

La phase aqueuse finale est dialysée pendant environ 60 heures contre 4 l d'eau distillée stérile et apyrogène.

Le dialysat est diafiltré sur membrane Amicon® $10^5$. On concentre à 50 ml. On ajoute 3 volumes d'éthanol absolu et de l'acétate de sodium (concentration finale 5 %). On laisse pendant une nuit à – 20 °C.

On recueille le précipité par centrifugation. Ce précipité est dissous dans de l'eau distillée stérile et apyrogène puis lyophilisé.

On obtient 1 050 g d'un produit qui contient :

| | |
|---|---|
| — sucres | 75 % |
| — protéines | 1,7 % |
| — acide nucléique | 0,7 % |

Toutes les opérations (sauf indication contraire) sont effectuées entre 0° et + 6 °C.

## Exemple 3

Polyoside pneumococcique du type 19.

5

On part d'une souche lyophilisée de Streptococcus pneumoniae du type 19.

On effectue la culture et la séparation du surnageant dans les conditions définies à l'exemple 1 (stades a à d).

L'extraction et la purification du polyoside sont effectuées de la façon suivante :

e) Extraction

En opérant comme à l'exemple 1-e, on concentre un volume de surnageant de 75 litres par trois diafiltrations à 2 litres. On obtient 2,3 g ayant la composition suivante :

| | |
|---|---|
| — sucres | 30 % |
| — protéines | 15 % |
| — acide nucléique | 3,4 % |

f) Purification

Le produit intermédiaire lyophilisé est dissous avec 150 ml de tampon phosphate 0,01 M — pH 7. On ajoute 3 mg de Dnase, 3 mg de RNase et 30 µl de RNase T1. On incube pendant 4 h à 37 °C. On ajoute ensuite 62 mg de Pronase, 150 µl de phénol et une goutte de toluène. On laisse agir pendant une nuit à 37 °C.

On déprotéinise par le mélange chloroforme-butanol 1 : 1, à volume égal (3 extractions successives).

La phase aqueuse est dialysée pendant 60 heures contre 4 litres d'eau distillée stérile et apyrogène. On effectue une diafiltration du dialysat sur membrane Amicon® XM 100.

On précipite le rétentat par 3 volumes d'éthanol contenant de l'acétate de sodium à 5 %.

Après une nuit au congélateur, le précipité est dissous dans l'eau stérile et apyrogène et lyophilisé. On obtient 730 mg de produit qui contient :

| | |
|---|---|
| — sucres | 73 % |
| — protéines | 0,2 % |
| — acide nucléique | 0,5 % |

Là encore, toutes les opérations (en dehors des incubations) sont effectuées entre 0 et + 4 °C.

Exemple 4

Polyoside pneumococcique du type 1.

On part d'une souche lyophilisée de Streptococcus pneumoniae du type 1. On effectue la culture et la séparation du surnageant dans les conditions définies à l'exemple 1.

L'extraction et la purification du polyoside sont effectuées de la façon suivante :

e) Extraction

100 litres de surnageant de culture sont diafiltrés sur cassettes Pellicon® (Millipore®, membrane $10^5$). Le volume du rétentat final est de 3 litres. On lyophilise en ballons. On obtient 6,1 g de produit contenant :

| | |
|---|---|
| — acide galacturonique | 46,5 % |
| — protéines | 7,7 % |

f) Purification

On dissout le produit obtenu dans 800 ml d'EDS (eau distillée stérile) apyrogène. On ajoute :

— 16 mg de RNase
— 16 mg de DNase
— 160 µl de RNase $T_1$.

On incube pendant 4 heures à 37 °C. On ajoute alors 300 µl de phénol, 2 gouttes de toluène et 150 mg de Pronase type 8. On incube pendant une nuit à 37 °C. On extrait encore les protéines à l'aide d'un mélange à volume égal de chloroforme-butanol (1 : 1). On dialyse la phase aqueuse pendant 60 heures. On diafiltre sur membrane Amicon® XM 100. Le volume de rétentat final est de 150 ml. On précipite la solution par 3 volumes d'éthanol froid contenant de l'acétate de sodium 5 %.

Le culot est repris par 250 ml d'EDS apyrogène. On lyophilise en flacons sous vide. On obtient 3,7 g de polyoside purifié contenant :

| | |
|---|---|
| — acide galacturonique | 55,5 % (ce qui correspond à 85 % de sucres) |
| — protéines | 1 % |

Le coefficient de diffusion dans le gel $K_D$ du polyoside est de 0,05. La masse moléculaire est donc d'au moins $2 \times 10^7$.

## 0 071 515

### Exemple 5

Polyoside de K. pneumoniae du type 2
On part d'une souche de Klebsiella pneumoniae du type 2.
On effectue la culture et la séparation du surnageant dans les conditions définies à l'exemple 1 (stade a à d), mais en utilisant comme milieu de culture le milieu de culture synthétique suivant :

| | |
|---|---|
| Citrate trisodique | 0,85 g |
| Sulfate d'ammonium | 0,17 g |
| Sulfate de magnésium | 0,17 g |
| Acide glutamique | 0,17 g |
| d-glucose | 16,70 g |
| Phosphate bipotassique | 10 g |
| Phosphate monopotassique | 6,66 g |
| Eau distillée q.s.p. | 1 000 ml |
| pH final : 6,8. | |

L'extraction et la purification du polyoside sont effectuées de la façon suivante :

e) Extraction
On utilise 90 litres de surnageant de culture. On diafiltre sur cassette Pellicon® (2 cassettes, membrane $10^5$). On effectue 3 lavages par 10 litres d'eau après avoir concentré à 1,5 litre. Le rétentat final est de 2,6 litres. On lyophilise en ballons, on obtient 6,1 g de produit qui contient :

| | |
|---|---|
| — sucres | 50 % |
| — protéines | 15 % |

f) Purification
Le produit obtenu est dissous dans 350 ml d'eau. On ajoute :

— 7 ml de tampon phosphate 0,5 M, pH 7,0
— 7 mg de RNase
— 7 mg de DNase
— 70 µl de Rnase $T_1$

(filtration préalable des enzymes sur 0,22 µ).
On incube 4 heures à 37 °C. On ajoute ensuite 70 mg de protéase type 8, 350 µl de phénol et 1 goutte de toluène. On incube pendant la nuit à 37 °C. On arrête la réaction et on achève la déprotéinisation par 3 extractions au butanol-chloroforme (1 : 1). On dialyse la phase aqueuse pendant 60 heures contre de l'EDS apyrogène. On diafiltre sur membrane Amicon® XM 100. Le volume de rétentat final est de 150 ml. On précipite la solution par 3 volumes d'éthanol absolu froid contenant de l'acétate de sodium 5 %. Le précipité, recueilli par centrifugation est dissous dans 250 ml d'eau, puis lyophilisé en flacons sous vide. On obtient un polyoside pur, contenant :

| | |
|---|---|
| — sucres | 76 % (Hexoses/acide uronique = 3 : 1) |
| — protéines | 3,8 % |

Le $K_D$ du polyoside obtenu est 0,1. La masse moléculaire est donc supérieure à $10^7$.

### Exemple 6

Polyoside de H. influenza du type b
On part d'une souche lyophilisée d'Hemophilus influenzae du type b.
On effectue la culture et la séparation du surnageant dans les conditions définies à l'exemple 1 (stade a à d) mais en utilisant comme milieu de culture le milieu de culture semi-synthétique suivant :

| | | |
|---|---|---|
| Proteose peptone | 5 | g |
| chlorure de sodium | 3,5 | g |
| d-glucose | 4 | g |
| Phosphate monopotassique | 1,3 | g |
| Phosphate bipotassique | 3,5 | g |
| NAD (nicotine adenosine dinucléotide) | 0,001 | g |
| Extrait globulaire | 50 | ml |
| Eau distillée q.s.p. | 1 000 | ml |

7

L'extraction et la purification du polyoside sont effectuées de la façon suivante :

e) Extraction

On utilise 30 litres de surnageant de culture. On effectue une diafiltration sur membrane Millipore® 100 000 (2 cassettes). La première concentration conduit à un volume de 1 litre. On lave par 3 fois 10 litres d'EDS. On concentre à 1,3 litre. On lyophilise en ballons. On obtient 3,9 g d'un produit qui contient :

| | |
|---|---|
| — sucres | 53 % |
| — protéines | 13 % |

Le produit obtenu est dissous dans 200 ml. On ajoute (après filtration sur 0,22 μ) :
4,5 ml de tampon phosphate 0,5 M, pH 7,0
4 mg RNase
4 mg DNase
40 μl RNase $T_1$

On effectue une incubation pendant 4 heures à 37 °C. Ensuite on ajoute 65 mg de protéase type 8, 200 μl de phénol et 2 gouttes de toluène. On incube pendant une nuit à 37 °C. On arrête la réaction par 3 extractions au butanol-chloroforme (1 : 1). On recueille la phase aqueuse par centrifugation à 12 000 t/min (17 000 g) à + 4 °C pendant 30 minutes. On dialyse pendant 60 heures contre de l'EDS apyrogène (changements de milieu de dialyse fréquents). On concentre sur membrane Amicon® XM 100. (2 lavages × 100 ml). Le volume du diafiltrat est de 100 ml. On précipite le diafiltrat par 3 volumes d'éthanol absolu froid contenant de l'acétate de sodium à 5 %. Le précipité, recueilli par centrifugation est repris dans 100 ml d'EDS apyrogène. La solution est lyophilisée en flacons sous vide.

On obtient 1,3 g de polyoside pur contenant :

| | |
|---|---|
| — * sucres (PRP) | 63 % |
| — protéines | 2 % |

Les polyosides capsulaires immunogènes purifiés obtenus selon la présente invention peuvent être utilisés pour la préparation de vaccins destinés à être administrés à titre préventif (ou curatif) à l'homme et aux animaux. Les vaccins peuvent être préparés par incorporation des polyosides capsulaires purifiés obtenus selon la présente invention dans un véhicule liquide physiologiquement acceptable, tel qu'une solution physiologique ou de l'eau pour injection.

On peut incorporer à cet effet un ou, de préférence, plusieurs polyosides capsulaires purifiés obtenus selon la présente invention et à partir de différents types sérologiques. En outre, des vaccins polyvalents peuvent être préparés par incorporation de deux ou plusieurs polyosides capsulaires purifiés obtenus à partir de différents germes.

On donnera ci-après des résultats des essais mettant en évidence l'activité immunogène des polyosides obtenus selon la présente invention.

a) Titrage des anticorps sériques chez la souris

α — Des souris mâles Swiss de 20 g ont été groupées par lots de 10 animaux :

1 lot témoin n'a reçu aucun traitement.

1 lot a été traité par voie I.P. à raison de plusieurs doses de polyoside de l'exemple 1 par animal.

Le titrage des anticorps hémagglutinants a été effectué selon les techniques décrites dans la littérature, utilisant le globule rouge de mouton sensibilisé par le polyoside à l'aide du chlorure de chrome.

Les résultats figurant dans le Tableau I mettent en évidence une augmentation du titre sérique des anticorps hémagglutinants chez les animaux traités aux faibles doses par rapport aux animaux témoins et une paralysie immunitaire classique pour les fortes doses.

β — Des essais analogues ont été effectués chez la souris avec le polyoside de l'exemple 5 (polyoside de Klebsiella pneumoniae, type 2).

Les résultats sont donnés dans le Tableau II.

* Le rapport des constituants du polyoside est le suivant : Ribose : Ribitol : Phosphore = 0,9 ; 1 ; 0,98 (rapport théorique : 1 : 1 : 1).

Le $K_D$ avoisine 0,1, ce qui correspond à une masse moléculaire d'environ $10^7$.

(Voir Tableaux I et II pages suivantes)

Tableau I

| | SOURIS – TITRES SERIQUES ANTICORPS HEMAGGLUTINANTS | | | | | | | |
|---|---|---|---|---|---|---|---|---|
| | Animaux témoins | | Animaux traités 77µg/souris | | Animaux traités 0,77µg/souris | | Animaux traités 0,077µg/souris | |
| | (1) | (2) | (1) | (2) | (1) | (2) | (1) | (2) |
| 1 | 128 | 7 | 0 | 0 | 64 | 6 | >4096 | 13 |
| 2 | 128 | 7 | 0 | 0 | 8 | 3 | 2048 | 12 |
| 3 | 128 | 7 | 0 | 0 | 512 | 9 | 128 | 7 |
| 4 | 16 | 4 | 0 | 0 | 32 | 5 | 128 | 7 |
| 5 | 4 | 2 | 0 | 0 | 512 | 9 | 256 | 8 |
| 6 | 8 | 3 | 0 | 0 | 256 | 8 | 128 | 7 |
| 7 | 64 | 6 | 0 | 0 | 256 | 8 | 256 | 8 |
| 8 | 64 | 6 | 0 | 0 | 1024 | 10 | 64 | 6 |
| 9 | 0 | 0 | 0 | 0 | 128 | 6 | 256 | 8 |
| 10 | 64 | 6 | 0 | 0 | 2048 | 11 | >4096 | 13 |
| | 4,8 $\pm$ 1,84 | | 0 | | 7,5 $\pm$ 1,85 | | 8,9 $\pm$ 2,02 | |
| p | | | | | < 0,05 | | < 0,01 | |
| (1) | Anticorps hémagglutinants inverse de la dilution | | | | | | | |
| (2) | Anticorps hémagglutinants $\log_2$ de l'inverse de la dilution | | | | | | | |

Tableau II

| | Animaux témoins | | Animaux traités 1630µg/kg | | Animaux traités 163µg/kg | | Animaux traités 16,3µg/kg | | Animaux traités 1,63µg/kg | |
|---|---|---|---|---|---|---|---|---|---|---|
| | (1) | (2) | (1) | (2) | (1) | (2) | (1) | (2) | (1) | (2) |
| 1 | 0 | | 0 | | 0 | | 8 | 3 | 0 | – |
| 2 | 0 | | 0 | | 0 | | 16 | 4 | 16 | 4 |
| 3 | 0 | | 0 | | 0 | | 32 | 5 | 16 | 4 |
| 4 | 0 | | 0 | | 0 | | 16 | 4 | 32 | 5 |
| 5 | 0 | | 0 | | 0 | | 8 | 3 | 64 | 6 |
| 6 | 0 | | 0 | | 8 | 3 | 16 | 4 | 32 | 5 |
| 7 | 0 | | 0 | | 0 | | 16 | 4 | 16 | 4 |
| 8 | 0 | | 0 | | 2 | 1 | 16 | 4 | 32 | 5 |
| 9 | 0 | | 0 | | 0 | | 32 | 5 | 64 | 6 |
| 10 | 0 | | Ü | | 0 | | 64 | 6 | 16 | 4 |
| | | | | | | | $4,2^{\pm}0,69$ | | $4,3^{\pm}1,28$ | |
| p | | | | | | | < 0,001 | | < 0,001 | |

(1)    Anticorps hémagglutinants − Inverse de la dilution

(2)    Anticorps hémagglutinants − $Log_2$ de l'inverse de la dilution

b) Titrage des anticorps sériques chez le rat.

La même étude a été réalisée chez le rat Sprague Dawley de 400-450 g mâle.

Les lots de 10 animaux ont reçu une injection I.P. de 2 et 20 µg de polyoside de Streptococcus pneumoniae, type 3, par animal.

Le titrage des anticorps hémagglutinants a été effectué les 4, 6 et 14e jours après l'administration.

Les résultats sont donnés dans le Tableau III.

En accord avec la littérature, le titre s'élève progressivement pour atteindre son taux maximal le 6e jour.

Tableau III

| | RATS - TITRES SERIQUES - ANTICORPS HEMAGGLUTINANTS $LOG_2$ DE L'INVERSE DE LA DILUTION | | | | | | | | |
|---|---|---|---|---|---|---|---|---|---|
| | Animaux témoins | | | Animaux traités 2µg/rat | | | Animaux traités 20µg/rat | | |
| | $J_4$ | $J_6$ | $J_{14}$ | $J_4$ | $J_6$ | $J_{14}$ | $J_4$ | $J_6$ | $J_{14}$ |
| 1 | 0 | 0 | 0 | 7 | 7 | 5 | 0 | 5 | 0 |
| 2 | 0 | 0 | 0 | 7 | 8 | 7 | 0 | 6 | 0 |
| 3 | 0 | 0 | 0 | 6 | 6 | 3 | 6 | 8 | 2 |
| 4 | 0 | 0 | 0 | 5 | 7 | 6 | 8 | 9 | 0 |
| 5 | 0 | 0 | 0 | 7 | 6 | 4 | 9 | 10 | 3 |
| 6 | 0 | 0 | 0 | 5 | 5 | 2 | 0 | 6 | 0 |
| 7 | 0 | 0 | 0 | 4 | 6 | 5 | 3 | 10 | 3 |
| 8 | 0 | 0 | 0 | 6 | 6 | 3 | 0 | 5 | 0 |
| 9 | 0 | 0 | 0 | 5 | 8 | 7 | 0 | 6 | 0 |
| | | | | 5,7 ± 0,89 | 6,55 ± 0,82 | 4,66 ± 1,46 | | 7,22 ± 1,66 | |
| p | | | | <0,001 | <0,001 | <0,001 | | <0,001 | NS |

c) Protection spécifique

α — Des lots de 10 souris mâles, d'origine Swiss ont été immunisés avec le polyoside de Streptococcus pneumoniae de type 1, obtenu selon l'exemple 4, par voie sous-cutanée (deux injections à une semaine d'intervalle) ; doses injectées : 0,47-4,7-47 et 470 µg/kg.

Le 10e jour après la 2e immunisation, les animaux ont été infectés avec le pneumocoque virulent du type 1, par voie sous-cutanée (1 250 germes/animal).

La mortalité après dix jours d'observation a été la suivante :

— animaux non traités     100 %
— animaux traités 0,47 µg/kg     30 %
— animaux traités 4,7 µg/kg     10 %
— animaux traités 47 µg/kg     30 %
— animaux traités 470 µg/kg     40 %

β — Des lots de 10 souris mâles, d'origine Swiss, ont été immunisés avec le polyoside de $KP_2$ par voie sous-cutanée (2 injections à une semaine d'intervalle) doses injectées : 53-533-5 332 µg/kg.

Le 8e jour après le dernier traitement, les animaux ont été injectés avec la souche de Klebsiella pneumoniae type 2 à raison de 650 germes par animal. La mortalité après 10 jours d'observation a été la suivante :

— animaux non traités     100 %
— animaux traités 53 µg/kg     0 %
— animaux traités 533 µg/kg     0 %
— animaux traités 5 333 µg/kg     0 %

Les polyosides obtenus selon la présente invention peuvent être utilisés notamment pour la préparation des vaccins suivants :

a) Vaccins destinés au traitement préventif et curatif des affections respiratoires aiguës et chroniques contenant un des polyosides purifiés correspondant à chacun des germes suivants :
Streptococcus pneumoniae
Hemophilus influenzae, type b
Klebsiella pneumoniae
Escherichia coli

A titre d'exemple

| 1) mélange de polyosides de S. pneumoniae, types 1, 6, 14, 23 | 50 µg |
| polyoside de K. pneumoniae, type 2 | 20 µg |
| polyoside d'H. influenzae, type b | 20 µg |

Volume final 0,5 ml d'eau physiologique

| 2) mélange de polyosides de S. pneumoniae, types 1, 6, 14, 23 | 25 µg |
| polyoside de K. pneumoniae, type 2 | 10 µg |
| polyoside d'H. influenza, type b | 10 µg |

b) Vaccin antipneumococcique contenant les polyosides purifiés des types suivants de Streptococcus pneumoniae :
1, 3, 4, 6A, 7F, 8, 9N, 12F, 14, 18L, 19F, 23, 2, 11A, 15F.
A titre d'exemples :
mélange à 50 µg, dans un volume de 0,5 ml d'eau physiologique,
mélange à 25 µg, dans un volume de 0,5 ml d'eau physiologique.


**Revendications** (pour les Etats contractants : BE, CH, DE, FR, GB, IT, LI, LU, NL, SE)

1. Procédé d'obtention de polyosides capsulaires immunogènes à partir de bactéries capsulées les contenant, caractérisé en ce que l'on effectue une culture de bactéries en milieu synthétique pouvant contenir jusqu'à environ 0,5 % en poids de produits d'origine naturelle, à la fin de la phase de croissance de cette culture on élimine les corps microbiens, on soumet la phase liquide débarrassée des corps microbiens à une séparation par filtration sur une membrane retenant les molécules d'une masse moléculaire égale ou supérieure à 100 000 daltons, obtenant ainsi un rétentat riche en polyosides capsulaires et l'on élimine les protéines, les acides nucléiques et les lipides de ce rétentat, obtenant ainsi un polyoside capsulaire purifié.

2. Procédé selon la revendication 1, caractérisé en ce que, pour éliminer les protéines, les acides nucléiques et les lipides du rétentat, on soumet le rétentat à une hydrolyse enzymatique, on ajoute un mélange butanol-chloroforme, on sépare la phase aqueuse, on soumet la phase aqueuse à une dialyse et on effectue une séparation par filtration sur une membrane retenant les molécules ayant une masse moléculaire supérieure à 100 000 daltons.

3. Procédé selon la revendication 1 ou la revendication 2, caractérisé en ce qu'on effectue la culture en milieu synthétique contenant jusqu'à 0,5 % en poids d'hydrolysats de protéine.

4. Procédé selon la revendication 3 pour l'obtention de polyosides capsulaires à partir de Streptococcus pneumoniae, caractérisé en ce que l'on utilise comme milieu de culture un milieu ayant sensiblement la composition suivante :

| Peptone pancréatique de caséine (IBF) | 1 500 | mg |
| l-cystine | 150 | mg |
| dl-tryptophane | 20 | mg |
| l-tyrosine | 200 | mg |
| Phosphate bipotassique | 4 960 | mg |
| d-glucose anhydre | 12 500 | mg |
| Sulfate de magnésium, $7H_2O$ | 500 | mg |
| Sulfate ferreux, $7H_2O$ | 5 | mg |
| Sulfate de zinc, $7H_2O$ | 0,8 | mg |
| Sulfate de manganèse, $7H_2O$ | 0,3 | mg |
| Acide chlorhydrique RP fumant | 17,8 | mg |
| d-biotine | 15 | mg |
| Acide nicotinique | 1 | mg |
| Pyridoxine HCl | 1 | mg |
| Riboflavine | 1 | mg |
| Thiamine | 1 | mg |
| Pentothénate de calcium | 5 | mg |

| | | |
|---|---|---|
| Adénine HCl | 10 | mg |
| Uracile | 10 | mg |
| Chlorure de choline | 10 | mg |
| Asparagine | 100 | mg |
| Eau distillée q.s.p. | 1 000 | ml |

5. Procédé selon la revendication 1 pour l'obtention de polyosides capsulaires à partir de Klebsiella pneumoniae, caractérisé en ce que l'on utilise comme milieu de culture un milieu ayant sensiblement la composition suivante :

| | | |
|---|---|---|
| Citrate trisodique | 0,85 | g |
| Sulfate d'ammonium | 0,17 | g |
| Sulfate de magnésium | 0,17 | g |
| Acide glutamique | 0,17 | g |
| d-glucose | 16,70 | g |
| Phosphate bipotassique | 10 | g |
| Phosphate monopotassique | 6,66 | g |
| Eau distillée q.s.p. | 1 000 | ml |
| pH final : 6,8. | — | |

6. Procédé selon la revendication 1 pour l'obtention de polyosides capsulaires à partir de Hemophilus influenzae, caractérisé en ce que l'on utilise comme milieu de culture un milieu ayant sensiblement la composition suivante :

| | | |
|---|---|---|
| Proteose peptone | 5 | g |
| Chlorure de sodium | 3,5 | g |
| d-glucose | 4 | g |
| Phosphate monopotassique | 1,3 | g |
| Phosphate bipotassique | 3,5 | g |
| NAD (nicotine adenosine dinucléotide) | 0,001 | g |
| Extrait globulaire | 50 | ml |
| Eau distillée q.s.p. | 1 000 | ml |

7. Procédé selon l'une quelconque des revendications 1 à 3, caractérisé en ce que l'on obtient les polyosides capsulaires à partir de Streptococcus pneumoniae, Hemophilus influenzae, Klebsiella pneumoniae ou Escherichia coli.

8. Vaccin contenant au moins un polyoside capsulaire obtenu par un procédé selon l'une quelconque des revendications 1 à 7.

**Revendications** (pour l'Etat contractant AT)

1. Procédé d'obtention de polyosides capsulaires immunogènes à partir de bactéries capsulées les contenant, caractérisé en ce que l'on effectue une culture de bactéries en milieu synthétique pouvant contenir jusqu'à environ 0,5 % en poids de produits d'origine naturelle, à la fin de la phase de croissance de cette culture on élimine les corps microbiens, on soumet la phase liquide débarrassée des corps microbiens à une séparation par filtration sur une membrane retenant les molécules d'une masse moléculaire égale ou supérieure à 100 000 daltons, obtenant ainsi un rétentat riche en polyosides capsulaires et l'on élimine les protéines, les acides nucléiques et les lipides de ce rétentat, obtenant ainsi un polyoside capsulaire purifié.

2. Procédé selon la revendication 1, caractérisé en ce que, pour éliminer les protéines, les acides nucléiques et les lipides du rétentat, on soumet le rétentat à une hydrolyse enzymatique, on ajoute un mélange butanol-chloroforme, on sépare la phase aqueuse, on soumet la phase aqueuse à une dialyse et on effectue une séparation par filtration sur une membrane retenant les molécules ayant une masse moléculaire supérieure à 100 000 daltons.

3. Procédé selon la revendication 1 ou la revendication 2, caractérisé en ce qu'on effectue la culture en milieu synthétique contenant jusqu'à 0,5 % en poids d'hydrolysats de protéine.

4. Procédé selon la revendication 3 pour l'obtention de polyosides capsulaires à partir de Streptococcus pneumoniae, caractérisé en ce que l'on utilise comme milieu de culture un milieu ayant sensiblement la composition suivante :

| | | |
|---|---|---|
| Peptone pancréatique de caséine (IBF) | 1 500 | mg |
| l-cystine | 150 | mg |
| dl-tryptophane | 20 | mg |
| l-tyrosine | 200 | mg |

13

| | | |
|---|---:|---|
| Phosphate bipotassique | 4 960 | mg |
| d-glucose anhydre | 12 500 | mg |
| Sulfate de magnésium, 7H$_2$O | 500 | mg |
| Sulfate ferreux, 7H$_2$O | 5 | mg |
| Sulfate de zinc, 7H$_2$O | 0,8 | mg |
| Sulfate de manganèse, 7H$_2$O | 0,3 | mg |
| Acide chlorhydrique RP fumant | 17,8 | mg |
| d-biotine | 15 | mg |
| Acide nicotinique | 1 | mg |
| Pyridoxine HCl | 1 | mg |
| Riboflavine | 1 | mg |
| Thiamine | 1 | mg |
| Pentothénate de calcium | 5 | mg |
| Adénine HCl | 10 | mg |
| Uracile | 10 | mg |
| Chlorure de choline | 10 | mg |
| Asparagine | 100 | mg |
| Eau distillée q.s.p. | 1 000 | ml |

5. Procédé selon la revendication 1 pour l'obtention de polyosides capsulaires à partir de Klebsiella pneumoniae, caractérisé en ce que l'on utilise comme milieu de culture un milieu ayant sensiblement la composition suivante :

| | | |
|---|---:|---|
| Citrate trisodique | 0,85 | g |
| Sulfate d'ammonium | 0,17 | g |
| Sulfate de magnésium | 0,17 | g |
| Acide glutamique | 0,17 | g |
| d-glucose | 16,70 | g |
| Phosphate bipotassique | 10 | g |
| Phosphate monopotassique | 6,66 | g |
| Eau distillée q.s.p. | 1 000 | ml |
| pH final : 6,8. | | |

6. Procédé selon la revendication 1 pour l'obtention de polyosides capsulaires à partir de Hemophilus influenzae, caractérisé en ce que l'on utilise comme milieu de culture un milieu ayant sensiblement la composition suivante :

| | | |
|---|---:|---|
| Proteose peptone | 5 | g |
| Chlorure de sodium | 3,5 | g |
| d-glucose | 4 | g |
| Phosphate monopotassique | 1,3 | g |
| Phosphate bipotassique | 3,5 | g |
| NAD (nicotine adenosine dinucléotide) | 0,001 | g |
| Extrait globulaire | 50 | ml |
| Eau distillée q.s.p. | 1 000 | ml |

7. Procédé selon l'une quelconque des revendications 1 à 3, caractérisé en ce que l'on obtient les polyosides capsulaires à partir de Streptococcus pneumoniae, Hemophilus influenzae, Klebsiella pneumoniae ou Escherichia coli.

**Claims** (for the Contracting States : BE, CH, DE, FR, GB, IT, LI, LU, NL, SE)

1. Process for the production of immunogenic capsular polyosides from capsulated bacteriae containing same, characterised in that it consists in cultivating the bacteriae in a synthetic medium which may contain up to about 0.5 % by weight of naturally occuring materials ; at the end of the growth stage of said culture removing the microbial bodies, submitting the liquid phase after removal of the microbial bodies to a filtration through a membrane which retains the molecules of a molecular weight of 100 000 daltons or more, to give a capsular polyosides-rich retained material ; and removing the proteins, the nucleic acids and the lipids from said retained material, to give a purified capsular polyoside.

2. Process as claimed in claim 1, characterised in that, to remove the proteins, the nucleic acids and the lipids from the retained material, said retained material is submitted to an enzyme hydrolysis, a butanol-chloroform mixture is added thereto, the aqueous phase is separated, said aqueous phase is submitted to a dialysis and then to a filtration through a membrane which retains the molecules having a molecular weight in excess of 100 000 daltons.

14

3. Process as claimed in claim 1 or 2, characterised in that the culture is effected in a synthetic medium comprising less than 0.5 wt % protein hydrolysates.

4. Process as claimed in claim 3 for the production of capsular polyosides from Streptococcus pneumoniae, characterised in that as culture medium is used a medium having substantially the following composition

| | | |
|---|---|---|
| Pancreatic peptone from casein (IBF) | 1 500 | mg |
| l-cystine | 150 | mg |
| dl-Tryptophane | 20 | mg |
| l-Tyrosine | 200 | mg |
| Dipotassium phosphate | 4 960 | mg |
| d-Glucose, anhydrous | 12 500 | mg |
| Magnesium sulfate. $7H_2O$ | 500 | mg |
| Ferrous sulfate. $7H_2O$ | 5 | mg |
| Zinc sulfate. $7H_2O$ | 0.8 | mg |
| Manganese sulfate. $7H_2O$ | 0.3 | mg |
| Fuming hydrochloric acid RP | 17.8 | mg |
| d-Biotine | 15 | mg |
| Nicotinic acid | 1 | mg |
| Pyridoxine HCl | 1 | mg |
| Riboflavine | 1 | mg |
| Thiamine | 1 | mg |
| Calcium pentothenate | 5 | mg |
| Adenine HCl | 10 | mg |
| Uracil | 10 | mg |
| Choline chloride | 10 | mg |
| Asparagine | 100 | mg |
| Distilled water, sufficient to make | 1 000 | ml |

5. A process as claimed in claim 1 for the production of capsular polyosides from Klebsiella pneumoniae, characterised in that as culture medium is used a medium having substantially the following composition

| | | |
|---|---|---|
| Trisodium citrate | 0.85 | g |
| Ammonium sulfate | 0.17 | g |
| Magnesium sulfate | 0.17 | g |
| Glutamic acid | 0.17 | g |
| d-Glucose | 16.70 | g |
| Dipotassium phosphate | 10 | g |
| Monopotassium phosphate | 6.66 | g |
| Distilled water, sufficient to make | 10 000 | ml |

6. A process as claimed in claim 1 for the preparation of capsular polyosides from Hemophilus influenzae, characterised in that as culture medium is used a medium having substantially the following composition

| | | |
|---|---|---|
| Proteose peptone | 5 | g |
| Sodium chloride | 3.5 | g |
| d-Glucose | 4 | g |
| Monopotassium phosphate | 1.3 | g |
| Dipotassium phosphate | 3.5 | g |
| NAD (nicotine adenosine dinucleotide) | 0.001 | g |
| Globular extract | 50 | ml |
| Distilled water, sufficient to make | 1 000 | ml |

7. Process as claimed in any one of claims 1-3 characterised in that capsular polyosides are obtained from Streptococcus pneumoniae, Hemophilus influenzae, Klebsiella pneumoniae or Escherichia coli.

8. Vaccine comprising at least one capsular polyoside produced by a process as claimed in any one of the claims 1-7.

**Claims** (for the Contracting State AT)

1. Process for the production of immunogenic capsular polyosides from capsulated bacteriae containing same, characterised in that it consists in cultivating the bacteriae in a synthetic medium which

15

0 071 515

may contain up to about 0.5 % by weight of naturally occuring materials ; at the end of the growth stage of said culture removing the microbial bodies, submitting the liquid phase after removal of the microbial bodies to a filtration through a membrane which retains the molecules of a molecular weight of 100 000 daltons or more, to give a capsular polyosides-rich retained material ; and removing the proteins, the nucleic acids and the lipids from said retained material, to give a purified capsular polyoside.

2. Process as claimed in claim 1, characterised in that, to remove the proteins, the nucleic acids and the lipids from the retained material, said retained material is submitted to an enzyme hydrolysis, a butanol-chloroform mixture is added thereto, the aqueous phase is separated, said aqueous phase is submitted to a dialysis and then to a filtration through a membrane which retains the molecules having a molecular weight in excess of 100 000 daltons.

3. Process as claimed in claim 1 or 2, characterised in that the culture is effected in a synthetic medium comprising less than 0.5 wt % protein hydrolysates.

4. A process as claimed in claim 3 for the production of capsular polyosides from Streptococcus pneumoniae, characterised in that as culture medium is used a medium having substantially the following composition

| | |
|---|---|
| Pancreatic peptone from casein (IBF) | 1 500 mg |
| l-cystine | 150 mg |
| dl-Tryptophane | 20 mg |
| l-Tyrosine | 200 mg |
| Dipotassium phosphate | 4 960 mg |
| d-Glucose, anhydrous | 12 500 mg |
| Magnesium sulfate. 7H$_2$O | 500 mg |
| Ferrous sulfate. 7H$_2$O | 5 mg |
| Zinc sulfate. 7H$_2$O | 0.8 mg |
| Manganese sulfate. 7H$_2$O | 0.3 mg |
| Fuming hydrochloric acid RP | 17.8 mg |
| d-Biotine | 15 mg |
| Nicotinic acid | 1 mg |
| Pyridoxine HCl | 1 mg |
| Riboflavine | 1 mg |
| Thiamine | 1 mg |
| Calcium pentothenate | 5 mg |
| Adenine HCl | 10 mg |
| Uracil | 10 mg |
| Choline chloride | 10 mg |
| Asparagine | 100 mg |
| Distilled water, sufficient to make | 1 000 ml |

5. A process as claimed in claim 1 for the production of capsular polyosides from Klebsiella pneumoniae, characterised in that as culture medium is used a medium having substantially the following composition

| | |
|---|---|
| Trisodium citrate | 0.85 g |
| Ammonium sulfate | 0.17 g |
| Magnesium sulfate | 0.17 g |
| Glutamic acid | 0.17 g |
| d-Glucose | 16.70 g |
| Dipotassium phosphate | 10 g |
| Monopotassium phosphate | 6.66 g |
| Distilled water, sufficient to make | 1 000 ml |

6. A process as claimed in claim 1 for the preparation of capsular polyosides from Hemophilus influenzae, characterised in that as culture medium is used a medium having substantially the following composition

| | |
|---|---|
| Proteose peptone | 5 g |
| Sodium chloride | 3.5 g |
| d-Glucose | 4 g |
| Monopotassium phosphate | 1.3 g |
| Dipotassium phosphate | 3.5 g |
| NAD (nicotine adenosine dinucleotide) | 0.001 g |
| Globular extract | 50 ml |
| Distilled water, sufficient to make | 1 000 ml |

16

7. Process as claimed in any one of claims 1-3 characterised in that capsular polyosides are obtained from Streptococcus pneumoniae, Hemophilus influenzae, Klebsiella pneumoniae or Escherichia coli.

**Patentansprüche** (für die Vertragsstaaten : BE, CH, DE, FR, GB, IT, LI, LU, NL, SE)

1. Verfahren zur Herstellung von immunogenen kapselförmigen Polyosiden aus sie enthaltenden Kapsel-Bakterien, dadurch gekennzeichnet, daß man ein Kultivierung von Bakterien im synthetischen Medium durchführt, das bis zu etwa 0,5 Gew.-% Produkte natürlichen Ursprungs enthalten kann, am Ende der Wachstumsphase dieser Kultivierung die Mikrobienkörper entfernt, die vom Mikrobienkörper befreite Flüssigphase einer Abtrennung durch Filtration auf einer Membran unterwirft, die die Moleküle mit einem Molekulargewicht von 100 000 Dalton oder mehr zurückhält, wobei man so ein an kapselförmigen Polyosiden reiches Retentat erhält, und die Proteine, die Nukleinsäuren und die Lipide von diesem Retentat entfernt und so ein gereinigtes kapselförmiges Polyosid erhält.

2. Verfahren nach Anspruch 1, dadurch gekennzeichnet, daß man zur Entfernung der Proteine, der Nukleinsäuren und der Lipide des Retentats das Retentat einer enzymatischen Hydrolyse unterwirft, ein Butanol-Chloroform-Gemisch zusetzt, die wässrige Phase abtrennt, die wässrige Phase einer Dialyse unterwirft und eine Trennung durch Filtration auf einer Membran durchführt, die die Moleküle mit einem Molekulargewicht von über 100 000 Dalton zurückhält.

3. Verfahren nach Anspruch 1 oder 2, dadurch gekennzeichnet, daß man die Kultivierung in synthetischem Medium durchführt, das bis zu 0,5 Gew.-% Proteinhydrolysate enthält.

4. Verfahren nach Anspruch 3 zur Herstellung von kapselförmigen Polyosiden aus Streptococcus pneumoniae, dadurch gekennzeichnet, daß man als Kulturmedium ein Medium mit im wesentlichen der folgenden Zusammensetzung verwendet :

| | | |
|---|---:|---|
| Pankreas-Pepton von Casein (IBF) | 1 500 | mg |
| l-Cystin | 150 | mg |
| dl-Tryptophan | 20 | mg |
| l-Tyrosin | 200 | mg |
| Dikaliumphosphat | 4 960 | mg |
| wasserfreie d-Glukose | 12 500 | mg |
| Magnesiumsulfat, $7H_2O$ | 500 | mg |
| Eisen-II-sulfat, $7H_2O$ | 5 | mg |
| Zinksulfat, $7H_2O$ | 0,8 | mg |
| Mangansulfat, $7H_2O$ | 0,3 | mg |
| rauchende RP Salzsäure | 17,8 | mg |
| d-Biotin | 15 | mg |
| Nicotinsäure | 1 | mg |
| Pyridoxinhydrochlorid | 1 | mg |
| Riboflavin | 1 | mg |
| Thiamin | 1 | mg |
| Calciumpentothenat | 5 | mg |
| Adeninhydrochlorid | 10 | mg |
| Uracil | 10 | mg |
| Cholinchlorid | 10 | mg |
| Asparagin | 100 | mg |
| destilliertes Wasser q.s.p. | 1 000 | ml |

5. Verfahren nach Anspruch 1 zur Herstellung von kapselförmigen Polyosiden aus Klebsiella pneumoniae, dadurch gekennzeichnet, daß man als Kulturmedium ein Medium mit im wesentlichen der folgenden Zusammensetzung verwendet :

| | | |
|---|---:|---|
| Trinatriumcitrat | 0,85 | g |
| Ammoniumsulfat | 0,17 | g |
| Magnesiumsulfat | 0,17 | g |
| Glutaminsäure | 0,17 | g |
| d-Glukose | 16,70 | g |
| Dikaliumphosphat | 10 | g |
| Monokaliumphosphat | 6,66 | g |
| destilliertes Wasser, q.s.p. | 1 000 | ml |
| End-pH : | 6,8 | |

6. Verfahren nach Anspruch 1 zur Herstellung von kapselförmigen Polyosiden aus Haemophilus influenzae, dadurch gekennzeichnet, daß man als Kulturmedium ein Medium mit im wesentlichen der folgenden Zusammensetzung verwendet :

| Proteosepepton | 5 | g |
|---|---|---|
| Natriumchlorid | 3,5 | g |
| d-Glukose | 4 | g |
| Monokaliumphosphat | 1,3 | g |
| Dikaliumphosphat | 3,5 | g |
| NAD (Nicotinadenosindinucleorid) | 0,001 | g |
| Glubolinextrakt | 50 | ml |
| destilliertes Wasser, q.s.p. | 1 000 | ml |

7. Verfahren nach irgendeinem der Ansprüche 1-3, dadurch gekennzeichnet, daß man die kapselförmigen Polyoside aus Streptococcus pneumoniae, Haemophilus influenzae, Klebsiella pneumoniae oder Escherichia coli erhält.

8. Vakzine, enthaltend wenigstens ein kapselförmiges Polyosid, erhalten nach einem Verfahren nach irgendeinem der Ansprüche 1 bis 7.

**Patentansprüche** (für den Vertragsstaat AT)

1. Verfahren zur Herstellung von immunogenen kapselförmigen Polyosiden aus sie enthaltenden Kapsel-Bakterien, dadurch gekennzeichnet, daß man eine Kultivierung von Bakterien im synthetischen Medium durchführt, das bis zu etwa 0,5 Gew.-% Produkte natürlichen Ursprungs enthalten kann, am Ende der Wachstumsphase dieser Kultivierung die Mikrobienkörper entfernt, die vom Mikrobienkörper befreite Flüssigphase einer Abtrennung durch Filtration auf einer Membran unterwirft, die die Moleküle mit einem Molekulargewicht von 100 000 Dalton oder mehr zurückhält, wobei man so ein an kapselförmigen Polyosiden reiches Retentat erhält, und die Proteine, die Nukleinsäuren und die Lipide von diesem Retentat entfernt und so ein gereinigtes kapselförmiges Polyosid erhält.

2. Verfahren nach Anspruch 1, dadurch gekennzeichnet, daß man zur Entfernung der Proteine, der Nukleinsäuren und der Lipide des Retentats das Retentat einer enzymatischen Hydrolyse unterwirft, ein Butanol-Chloroform-Gemisch zusetzt, die wässrige Phase abtrennt, die wässrige Phase einer Dialyse unterwirft und eine Trennung durch Filtration auf einer Membran durchführt, die die Moleküle mit einem Molekulargewicht von über 100 000 Dalton zurückhält.

3. Verfahren nach Anspruch 1 oder 2, dadurch gekennzeichnet, daß man die Kultivierung in synthetischem Medium durchführt, das bis zu 0,5 Gew.-% Proteinhydrolysate enthält.

4. Verfahren nach Anspruch 3 zur Herstellung von kapselförmigen Polyosiden aus Streptococcus pneumoniae, dadurch gekennzeichnet, daß man als Kulturmedium ein Medium mit im wesentlichen der folgenden Zusammensetzung verwendet :

| Pankreas-Pepton von Casein (IBF) | 1 500 | mg |
|---|---|---|
| l-Cystin | 150 | mg |
| dl-Tryptophan | 20 | mg |
| l-Tyrosin | 200 | mg |
| Dikaliumphosphat | 4 960 | mg |
| wasserfreie d-Glukose | 12 500 | mg |
| Magnesiumsulfat, $7H_2O$ | 500 | mg |
| Eisen-II-sulfat, $7H_2O$ | 5 | mg |
| Zinksulfat, $7H_2O$ | 0,8 | mg |
| Mangansulfat, $7H_2O$ | 0,3 | mg |
| rauchende RP Salzsäure | 17,8 | mg |
| d-Biotin | 15 | mg |
| Nicotinsäure | 1 | mg |
| Pyridoxinhydrochlorid | 1 | mg |
| Riboflavin | 1 | mg |
| Thiamin | 1 | mg |
| Calciumpentothenat | 5 | mg |
| Adeninhydrochlorid | 10 | mg |
| Uracil | 10 | mg |
| Cholinchlorid | 10 | mg |
| Asparagin | 100 | mg |
| destilliertes Wasser q.s.p. | 1 000 | ml |

5. Verfahren nach Anspruch 1 zur Herstellung von kapselförmigen Polyosiden aus Klebsiella pneumoniae, dadurch gekennzeichnet, daß man als Kulturmedium ein Medium mit im wesentlichen der folgenden Zusammensetzung verwendet :

| | |
|---|---|
| Trinatriumcitrat | 0,85 g |
| Ammoniumsulfat | 0,17 g |
| Magnesiumsulfat | 0,17 g |
| Glutaminsäure | 0,17 g |
| d-Glukose | 16,70 g |
| Dikaliumphosphat | 10 g |
| Monokaliumphosphat | 6,66 g |
| destilliertes Wasser, q.s.p. | 1 000 ml |
| End-pH : | 6,8 |

6. Verfahren nach Anspruch 1 zur Herstellung von kapselförmigen Polyosiden aus Haemophilus influenzae, dadurch gekennzeichnet, daß man als Kulturmedium ein Medium mit im wesentlichen der folgenden Zusammensetzung verwendet :

| | |
|---|---|
| Proteosepepton | 5 g |
| Natriumchlorid | 3,5 g |
| d-Glukose | 4 g |
| Monokaliumphosphat | 1,3 g |
| Dikaliumphosphat | 3,5 g |
| NAD (Nicotinadenosindinucleorid) | 0,001 g |
| Glubolinextrakt | 50 ml |
| destilliertes Wasser, q.s.p. | 1 000 ml |

7. Verfahren nach irgendeinem der Ansprüche 1-3, dadurch gekennzeichnet, daß man die kapselförmigen Polyoside aus Streptococcus pneumoniae, Haemophilus influenzae, Klebsiella pneumoniae oder Escherichia coli erhält.